# EUROPEAN PATENT APPLICATION

(11) **EP 1 634 561 A1**
(43) Date of publication of application: **15.03.2006**
(21) Application number: 04018746.0
(22) Date of filing: 06.08.2004
(51) Int. Cl.: A61K 6/083

(54) **Reactive filler for dental cements**

(71) Applicant: DENTSPLY DETREY GmbH, 78467 Konstanz (DE)
(72) Inventor: Brugger, Stefan, 78315 Radolfzell (DE); Schellong, Alice, 78048 Villingen-Schwenningen (DE)
(74) Representative: Hartz, Nikolai

(57) **Abstract**

A process for the modification of a particulate reactive filler for a dental ionomer cement, comprising
(a) providing a particulate reactive filler; and
   (b1) treating the surface of the particulate reactive filler with a surface modifying agent for obtaining a surface modified particulate reactive filler displaying ligand groups for a transition metal; and
   (b2) treating the surface modified particulate reactive filler with an agent containing the transition metal for complexing the transition metal with the ligand groups displayed on the surface of the surface modified particulate reactive filler; and/or
(c) treating the surface of the particulate reactive filler with a surface modifying transition metal complex which is a reaction product of a surface modifying agent and a transition metal precursor compound,
for obtaining a transition metal complex surface modified particulate reactive filler for a dental ionomer cement.

## Description

### Field of the invention

The present invention relates to a process for the modification of a reactive particulate filler for a dental ionomer cement. Moreover, the present invention relates to a modified reactive particulate filler for a dental ionomer cement obtainable by the process of the present invention. Furthermore, the present invention relates to a dental ionomer cement comprising the reactive particulate filler of the invention. Finally, the present invention provides a use of the reactive particulate filler in a dental ionomer cement.

### Background of the invention

lonomer cements are known. lonomer cements commonly contain a polycarboxylic acid and an inorganic powder which react in the presence of water by a curing reaction. Conventional ionomer cements generally contain a powder component containing aluminosilicate and a liquid portion usually containing a polyacid such as polyacrylic acid, polymaleic acid, polyitaconic acid, or a copolymer of at least two of the acids, cf. "New Aspects of the Setting of Glass-ionomer Cements," Wasson et al., Journal of Dental Research; Vol. 72, No. 2, February, 1993; pages 481-483. In glass ionomer cements, the primary reactions which cause the glass ionomer cement to harden is cross-linking of polycarboxylate chains by metal ions from the glass based on ionic forces. Moreover, during setting the acids of the glass ionomer cement dissolve the glass structure to release metal constituents of the glass. Ionic carboxylates of calcium and strontium are mainly formed during the setting process.

lonomer cements are characterized by good adhesion properties to enamel and dentin, good aesthetic properties, and the possibility for anticariogenic properties due to the release of fluoride from a fluoride containing glass filler. Accordingly, ionomer cements are widely used in the dental field for filling of a caries cavity, cementing of crowns, inlays, bridges, or orthodontic bands, lining of a cavity, sealing of a root canal, core construction, and preventive sealing.

However, the mechanical properties of ionomer cements are usually problematic: glass ionomer materials are inherently brittle. Therefore, the application of ionomer cements is usually limited to non-stress bearing areas. lonomer cement materials continue to have significant limitations for use in permanent posterior, particularly with regard to large restorations.

Resin-modified glass-ionomer cements were introduced with an aim of overcoming the problems associated with the tendency towards brittle fracture of conventional glass-ionomer, while still retaining advantages such as fluoride release and adhesion, EP 0323120, US-A 4,872,936 and US-A 5,154,762. Accordingly, it was suggested to replace some of the water in a conventional glass-ionomer cement with a hydrophilic monomer or to modify the polymeric acid so that some of the acid groups were replaced with polymerisable moieties, so that the polymeric acid could also take part in a polymerisation reaction.

Moreover, in order to address the problem of improving the mechanical properties of ionomer cement materials, US-A 5,369,142 suggests the use of a specific acidic component, namely copolymers of acryloyl or methacryloyl derivatives of amino acids with acrylic acid or methacrylic acid. WO-A 02/062861 discloses polymer compositions for use in glass ionomer dental restoratives having improved resistance to bending and resistance to twisting, whereby the polymers are formed from at least two specific polymers. WO-A 03/061606 discloses ionomer cements containing amino acids improving the mechanical properties.

### Summary of the invention

It is a problem of the invention to provide a ionomer cement having improved mechanical properties, in particular improved compressive strength and flexural strength, while at the same time having excellent working and setting times.

It is a further problem of the present invention to provide a modified reactive glass filler for a ionomer cement having improved mechanical properties, in particular improved compressive strength and flexural strength.

It is a further problem of the invention to provide a process for the modification of a reactive particulate filler for a dental ionomer cement.

It is a still further problem of the present invention to provide a use of the reactive particulate filler obtained according to the present invention.

The present invention provides a process for the modification of a reactive particulate filler for a dental ionomer cement, comprising
(a) providing a particulate reactive filler; and
   (b1) treating the surface of the particulate reactive filler with a surface modifying agent for obtaining a surface modified particulate reactive filler displaying ligand groups for a transition metal; and
   (b2) treating the surface modified particulate reactive filler with an agent containing the transition metal for complexing the transition metal with the ligand groups displayed on the surface of the surface modified particulate reactive filler; and/or
   (c) treating the surface of the particulate reactive filler with a surface modifying transition metal complex which is a reaction product of a surface modifying agent and a transition metal precursor compound,
for obtaining a transition metal complex surface modified particulate reactive glass for a dental ionomer cement.

The present invention is based on the recognition that ionic salt bridges at interfaces between the particulate reactive filler and the acidic component formed during the curing of a conventional ionomer cement are inherently insufficient for providing good mechanical properties, in particular improved compressive strength and flexural strength, of the glass ionomer material. The present invention is furthermore based on the recognition that the interfacial bonding between the acidic component and a particulate reactive filler may be improved by a surface modification of the particulate reactive filler. According to the present invention, interfacial bonding between a particulate reactive filler and the acidic component is provided by a combination of conventional ionic salt bridges and the interaction of a transition metal complexed by ligands displayed by the particulate reactive filler and a polyacidic polymer.

### Detailed description of the invention

The present invention provides a process for the modification of a reactive particulate filler for a dental ionomer cement. The process comprises the step of providing a particulate reactive filler. A "particulate reactive filler" is a powdered metal oxide or hydroxide, mineral silicate, or ion leachable glass or ceramic, that is capable of reacting with an ionomer in the presence of water to form a hydrogel. Examples of particulate reactive filler materials include materials commonly known in the art of glass-ionomer cements such as calcium or strontium-containing and aluminum-containing materials. Preferably, particulate reactive fillers contain leachable fluoride ions. Specific examples of particulate reactive fillers are selected from calcium alumino silicate glass, calcium alumino fluorosilicate glass, calcium aluminumfluoroborosilicate glass, strontium aluminosilicate glass, strontium aluminofluorosilicate glass, strontium aluminofluoroborosilicate glass. Suitable particulate reactive fillers further include metal oxides such as zinc oxide and magnesium oxide, and ion-leachable glasses, e.g., as described in US-A 3,655,605, US-A 3,814,717, US-A 4,143,018, US-A 4,209,434, US-A 4,360,605 and US-A 4,376,835.

The particulate reactive filler usually has an average particle size of from 0.005 to 100 µm, preferably of from 0.01 to 40 µm as measured using, for example, by electron microscopy or by using a conventional laser diffraction particle sizing method as embodied by a MALVERN Mastersizer S or MALVERN Mastersizer 2000 apparatus. The particulate reactive filler may be a multimodal particulate reactive filler representing a mixture of two or more particulate fractions having different average particle sizes. The particulate reactive filler may also be a mixture of particles of different chemical composition. In particular, it is possible to use a mixture of a particulate reactive material and a particulate non-reactive material.

The process further comprises a step of surface modification for obtaining a transition metal complex surface modified reactive particulate filler for a dental ionomer cement. The surface modification may be achieved by treating the surface of the particulate glass ionomer with a surface modifying agent for obtaining a surface modified particulate glass ionomer displaying ligand groups for a transition metal and treating the surface modified particulate glass ionomer with an agent containing the transition metal for complexing the transition metal with the ligand groups displayed on the surface of the surface modified particulate glass ionomer.

Alternatively or additionally, the surface modification may be achieved by treating the surface of the particulate glass ionomer with a surface modifying transition metal complex which may be a reaction product of a surface modifying agent and a transition metal.

In the process of the present invention, the surface modifying agent contains a modifying compound providing a dual function. The modifying compound is capable of reacting with surface atoms of the particulate reactive filler, thereby forming a covalent bond between the surface atoms of the particulate reactive filler and the modifying compound. Moreover, the modifying compound contains one or more heteroatoms capable of complexing a transition metal ion, thereby attaching a transition metal ion to the surface of the particulate reactive filler. The modifying agent may contain one or more modifying compounds. Preferably, the modifying compound provides a chelating ligand capable of complexing a transition metal with two or more hetero atoms.

Preferably, the surface modifying agent contains a hydrolyzable organofunctional silicon compound. The hydrolyzable organofunctional silicon compound may be a compound of one of the following formulae (I), (II) and (III), or a hydrolysis product thereof

XₙR₃₋ₙSiL (I)

XₙR₂₋ₙSiL'L" (II)

XₙSiL'L"L"' (III)

wherein
- X: represents a hydrolyzable group;
- R: represents an alkyl, cycloalky, cycloalkylalkyl, aralkyl or aryl group,
- L, L', L", and L"': which may be the same or different represent independent from each other an organic group containing hetero atoms capable of coordinating to the transition metal;
- n: is an integer ≥ 1,
whereby the sum of X, R, L, L', L", and L"' is 4 for each of formula (I), (II), and (III).

Preferably, X is a halogen atom or OR¹, wherein R¹ is an alkyl, cycloalky, cycloalkylalkyl, aralkyl or aryl group. More preferably, R or R¹ are independently an alkyl group.

In order to impart complexing capability to the organofunctional silicon compound, L, L', L", and L"' contain heteroatoms such as nitrogen atoms, oxygen atoms, sulfur and/or phosphorous atoms capable of binding to the transition metal. In a preferred embodiment, L, L', L", and L"' may be represented by the following formula:

-[(CH₂)ₒZ]_{q}(CH₂)ₚZR'

wherein
the Zs which may be the same or different and are independent from each other, represent - NR'- , -O-, S or PR', wherein R' represents independently a hydrogen atom, an alkyl group, a cycloalkyl group, an cycloalkylalkyl group, an aralkyl group or an aryl group,
o and p, which are independent from each other, may be the same or different and represent an integer of from 1 to 6, and
q represents an integer of from 0 to 12.

In a further preferred embodiment, L, L', L", and L"' may be represented by the following formula:

-[(CH₂)ₒNR']_{q}(CH₂)ₚNR"R"'

wherein
R', R" and R"', which are independent from each other, may be the same or different and represent a hydrogen atom, an alkyl group, a cycloalkyl group, an cycloalkylalkyl group, an aralkyl group or an aryl group,
o and p, which are independent from each other, may be the same or different and represent an integer of from 1 to 6, and
q represents an integer of from 0 to 12.

In a still further preferred embodiment, L, L', L", and L"' may be represented by the following formula:

-[(CH₂)ₒZ]_{q}(CH₂)ₚZR'

wherein
R' represents a hydrogen atom, an alkyl group, a cycloalkyl group, an cycloalkylalkyl group, an aralkyl group or an aryl group,
Z represents an oxygen atom or a sulfur atom,
o and p, which are independent from each other, may be the same or different and represent an integer of from 1 to 6, and
q represents an integer of from 0 to 12.

An alkyl group may be straight-chain or branched C₁₋₁₆ alkyl group, typically a C₁₋₈ alkyl group. Examples for a C₁₋₆ alkyl group can include linear or branched alkyl groups having 1 to 6 carbon atoms, preferably 1 to 4 carbon atoms, for example, methyl, ethyl, n-propyl, isopropyl, n-butyl, isobutyl, sec-butyl, tert-butyl, n-pentyl, isopentyl and n-hexyl. A cycloalkyl group may be a C₃₋₁₆ cycloalkyl group. Examples of the cycloalkyl group can include those having 3 to 14 carbon atoms, for example, cyclopropyl, cyclobutyl, cyclopentyl and cyclohexyl. A cycloalkylalkyl group can include those having 4 to 22 carbon atoms. Examples for a cycloalkylalkyl group can include a combination of a linear or branched alkyl group having 1 to 6 carbon atoms and a cycloalkyl group having 3 to 14 carbon atoms. Examples of the cycloalkylalkyl group can for example, include methylcyclopropyl, methylcyclobutyl, methylcyclopentyl, methylcyclohexyl, ethylcyclopropyl, ethylcyclobutyl, ethylcyclopentyl, ethylcyclohexyl, propylcyclopropyl, propylcyclobutyl, propylcyclopentyl, propylcyclohexyl. An aralkyl group may be a C₇₋₂₆ aralkyl group, typically a combination of a linear or branched alkyl group having 1 to 6 carbon atoms and an aryl group having 6 to 10 carbon atoms. Specific examples of an aralkyl group are a benzyl group or a phenylethyl group. An aryl group can include aryl groups having 6 to 10 carbon atoms. Examples of the aryl group are phenyl and naphtyl.

The C₁₋₆ alkyl group and the C₃₋₁₄ cycloalkyl group may optionally be substituted by one or more members of the group selected from a C₁₋₄ alkyl group, C₁₋₄ alkoxy group, a phenyl group, and a hydroxy group. Examples for a C₁₋₄ alkyl group can include linear or branched alkyl groups having 1 to 4 carbon atoms, for example, methyl, ethyl, n-propyl, isopropyl, n-butyl, isobutyl, sec-butyl, tert-butyl. Examples for an C₁₋₄ alkoxy group can include linear or branched alkoxy groups having 1 to 4 carbon atoms, for example, methoxy, ethoxy, n-propoxy, isopropoxy, n-butoxy, isobutoxy, sec-butoxy, and tert-butoxy.

Aryl groups may contain 1 to 3 substituents. Examples of such substituents can include halogen atoms, C₁₋₄ alkyl groups, C₁₋₄ alkoxy groups, C₁₋₄ alkylthio groups, C₁₋₄ alkylsulfonyl groups, carboxyl group, C₂₋₅ alkoxycarbonyl groups, and C₁₋₄ alkylamino groups. Here, illustrative of the halogen atoms can be fluorine, chlorine, bromine and iodine. The C₁₋₄ alkyl groups are, for example, methyl, ethyl, n-propyl, isopropyl and n butyl. Illustrative of the C₁₋₄ alkoxy groups are, for example, methoxy, ethoxy and propoxy. Illustrative of the C₁₋₄ alkylthio groups are, for example, methylthio, ethylthio and propylthio. Illustrative of the C₁₋₄ alkylsulfonyl groups are, for example, methylsulfonyl, ethylsulfonyl and propylsulfonyl. Illustrative of the C₂₋₅ alkoxycarbonyl groups can be those having alkoxy groups each of which contains 1 to 4 carbon atoms, for example, methoxycarbonyl, ethoxy carbonyl and propoxycarbonyl. Illustrative of the C₁₋₈ alkylamino groups can be those having one or two alkyl groups each of which contains 1 to 4 carbon atoms, for example, methylamino, dimethylamino, ethyl amino and propylamino. The alkyl moieties in these substituents may be linear, branched or cyclic.

Specific examples of modifying compounds contained in the surface modifying agent used in the present invention are aminopropyltrimethoxysilane, aminopropylmethyldimethoxysilane, aminopropyldimethylmethoxysilane, aminopropyltriethoxysilane (APTES), aminopropylmethyldiethoxysilane, aminopropyldimethylethoxysilane, 2-(aminoethyl)-3-aminopropyltrimethoxysilane (AEPTMS), 2-(aminoethyl)-3-aminopropyldimethoxymethylsilane, 2-(aminoethyl)-3-aminopropyldimethylmethoxysilane, 2-(aminoethyl)-3-aminopropyltriethoxysilane, 2-(aminoethyl)-3-aminopropyldiethoxymethylsilan, 2-(aminoethyl)-3-aminopropyldimethylethoxysilane, (3-trimethoxysilylpropyl)diethylenetriamine,(3-dimethoxymethylsilylpropyl)diethylenetriamine,(3-dimethylmethoxysilylpropyl)diethylenetriamine, (3-triethoxysilylpropyl)diethylenetriamine (TMSPDETA), (3-diethoxymethylsilylpropyl)diethylenetriamine, (3-dimethylethoxysilylpropyl)diethylenetriamine. The compounds may be used alone or in combination of two or more different compounds.

Based on the treatment of the particulate reactive filler with the surface active agent, the surface of the reactive filler displays ligand groups capable of complexing a transition metal. The ligand groups correspond for example to the groups L, L', L", and L"' as described above. The ligand groups may be monodentate ligand groups or chelating ligand groups.

The surface modifying agent may be used as such or dissolved or dispersed in a suitable solvent. Examples of suitable solvent are toluene, methanol, ethanol, isopropanol, and ethylacetate.

Subsequent to the surface modification of the surface of the particulate reactive filler, the surface modified particulate reactive filler is treated with an agent containing the transition metal for complexing the transition metal with the ligand groups displayed on the surface of the surface modified particulate reactive filler.

Preferred transition metals are scandium, yttrium, lanthanum, cerium, praseodymium, neodymium, samarium, europium, gadolinium, terbium, dysprosium, holmium, erbium, thulium, ytterbium, lutetium, manganese, copper, silver, ruthenium, rhodium, palladium, zinc and iron.

A more preferred group of transition metals is yttrium, lanthanum, cerium, samarium, europium, gadolinium, terbium, holmium, ytterbium, lutetium, copper, and zinc.

The agent containing the transition metal may contain a single transition metal or a combination of two or more transition metals. The transition metal may be in the form of an ion, preferably in the form of an ion which is acceptable for dental purposes and a the same time capable of forming a complex with a carboxyl group. The transition metal contained in the agent may be derived from a transition metal precursor component. The transition metal precursor component may be a soluble salt such as a halogenide, sulfate, carbonate, or carboxylate such as an acetate.

The agent containing the transition metal may further contain a suitable solvent. The solvent may be selected from aqueous or organic solvents or mixtures thereof. Suitable organic solvents are alcohols, ethers, ketones, hydrocarbons, of halogenated hydrocarbons.

Alternatively or additionally, the surface modification of the particulate reactive filler may be achieved by treating the surface of the particulate glass ionomer with a surface modifying transition metal complex. The transition metal complex is a reaction product of a surface modifying agent and a transition metal precursor compound. The modifying agent may contain one or more modifying compounds. The modifying compound contains one or more heteroatoms capable of complexing a transition metal ion. Moreover, the surface modifying agent contains a modifying compound capable of reacting with surface atoms of the particulate reactive filler. Accordingly, the surface modifying transition metal complex may be linked to the surface of the particulate reactive filler by a covalent bond between the surface of the particulate reactive filler and the modifying compound.

The modifying agent used for the preparation of the surface modifying transition metal complex may be the same modifying agent as described above. Specifically, the modifying agent may contain one or more modifying compounds as described above. The modifying compounds may be used as such or dissolved or dispersed in a suitable medium such as toluene, methanol, ethanol, isopropanol, and ethylacetate.

The above surface modifcation provides a transition metal complex surface modified reactive particulate filler for a dental ionomer cement. A dental ionomer cement comprises a modified reactive particulate filler obtainable according to the process of the present invention and an ionomer. An ionomer contains a polymer having sufficient pendent ionic groups to undergo a setting reaction or curing reaction in the presence of the modified reactive filler material and water. As used herein, the term "polymer" includes molecules whose backbone is derived from one monomer (viz. a homopolymer) or from two or more monomers (viz., a copolymer). A polymer typically has a weight average molecular weight of at least about 2000. The ionomer may further contain monomeric acids or monomers capable of polymerising in the presence of an initiator system activated by light or a redox reaction, thereby further curing the cement. Water serves as a medium facilitating the transport of ions between the ionomer and the filler, thereby allowing the acid-base chemical cure setting reaction to occur. In the present invention, the pendent ionic groups of the ionomer may also react with the transition metal thereby forming a transition metal complex comprising the transition metal, the ligand groups of the surface modifying compound covalently linked to the surface of the particulate reactive filler and the pendent ionic groups of the ionomer.

Polymerizable acids used for preparing ionomer polymers useful for glass-ionomer cement systems include alkenoic acids and unsaturated mono-, di-and tricarboxylic acids. Representative alkenoic acids are described, for example, in US-A 4,016, 124, US-A 4,089, 830, US-A 3,655, 605; US-A 4,143, 018; US-A 4,342, 677, US-A 4,360,605, US-A 4,376, 835 and US-A 5,130, 347. Specific examples are acrylic acid, methacrylic acid, maleic acid, fumaric acid, itaconic acid, crotonic acid, and derivatives thereof, such as the acid chlorides thereof, the acid anhydrides thereof and chloro or bromo derivatives thereof. Particularly preferred monomers are acrylic acid, itaconic acid and maleic acid, and the chlorides or anhydrides thereof. The pendent carboxylic acid groups of the ionomer must be sufficient in number or percent by weight to bring about the setting or curing reaction in the presence of the modified particulate reactive filler.

It is possible to create a source of additional covalent cross- linking, which imparts additional strength to the ultimate ionomeric cement composition, by reacting a portion of the carboxylic acid groups with a bi-functional monomer. Examples of suitable bi-functional monomers include acryloyl chloride, methacryloyl chloride, vinyl azalactone, allyl isocyanate, 2-hydroxyethylmethacrylate (HEMA), 2-aminoethylmethacrylate, 2-isocyanatoethyl methacrylate (IEM), acrylic acid, methacrylic acid and N-vinylpyrrolidone. Other examples of suitable bi-functional monomers are described in US-A 4,035, 321 US-A 5,130, 347. By adding a polymerizable resin component to the ionomer cement, not only the brittleness may be further improved, but also the mechanical strengths and physical properties such as adhesiveness to a tooth structure are improved. In addition, ionomer cements, which can be quickly cured by visible light by using a photopolymerization catalyst as a catalyst for polymerizing a polymerizable resin component, may be provided.

To effect cross-linking or additional cross-linking of the cement, one or more comonomers may be included in the cement composition. Suitable comonomers contains at least one polymerizable functional group. Suitable polymerizable functional groups are ethylenically unsaturated groups (e. g. , alkenyl groups and preferably vinyl groups). Ethylenically unsaturated groups are polymerisable by a free radical mechanism. Preferred examples are substituted and unsubstituted acrylates, methacrylates, or alkenes.

Methods for preparing the ionomers are well known. (Crisp et al. , "Glass ionomer cement formulations. II. The synthesis of novel polycarboxylic acids,"in J. Dent. Res. 59 (6) : 1055-1063 (1980)). A dental ionomer cement is prepared by mixing the ionomer with the modified particulate reactive filler in the presence of water. The components of the ionomer cement system can be combined (such as by mixing or blending) in a variety of manners and amounts in order to form the ionomer cements of the present invention. For example, a concentrated aqueous solution of the ionomer may be mixed with the modified particulate reactive filler and optionally further components at the time of use. The resultant combination of ionomer, modified particulate reactive filler and water allows the setting reaction to begin. Alternatively, the ionomer and the modified particulate reactive filler are provided as a freeze-dried or lyophilized powdered blend under conditions in which there is not sufficient water to allow the setting reaction to proceed. Such systems can then be combined with water at the time of use in order to begin the setting reaction. Once the setting reaction has begun, the resultant mixture may be formed into its desired shape, followed by curing and allowing the mixture to fully harden. In general, the weight-to-weight ratio of the ionomer to water is from about 1: 10 to about 10: 1. In general, the concentration of ionomer in water ranges from 25 to 75% by weight, and preferably from 40 to 65 percent. The resultant aqueous solution has a ratio of polymer to liquid generally ranging from about 1.5 to 8.

The reaction mixture may also include a modifying agent such as tartaric acid, for adjusting the working time and a setting time, respectively, when preparing the cement as described in US-A 4,089, 830, US-A 4, 209,434, US-A 4,317, 681 and US-A 4,374, 936. In general, an increase in working time results in an increase in setting time as well. The "working time"is the time between the beginning of the setting reaction when the ionomer and modified particulate reactive filler are combined in the presence of water, and the time the setting reaction proceeds to the point when it is no longer practical to perform further physical work upon the system, e.g. spatulate it or reshape it, for its intended dental or medical application. The "setting time" is the time measured from the beginning of the setting reaction in a restoration to the time sufficient hardening has occurred to allow subsequent clinical or surgical procedures to be performed on the surface of the restoration.

In the setting reaction, the modified particulate reactive filler behaves like a base and reacts with the acidic ionomer to form a metal polysalt which acts as the binding matrix (Prosser, J. Chem. Tech. Biotechnol. 29 : 69-87(1979)). Moreover, due to the presence of transition metal complexes having coordination sites available for ligand exchange or further coordination, a reaction between the ionomer and the transition metal complexes covalently attached to the surface of the modified particulate reactive filler takes place. Thereby the bonding of the ionomer to the modified particulate reactive filler does not only rely on ionic salt bridges which are problematic with regard to the mechanical properties, but also on covalent and complex bonding. The setting reaction is therefore characterized as a dual chemical cure system that proceeds automatically upon mixing the ionomer and modified particulate reactive filler material in the presence of water. The cement sets to a gel-like state within a few minutes and rapidly hardens to develop strength.

The ratio of powder to liquid affects the workability of the mixed ionomer cement systems. Weight ratios higher than 20:1 tend to exhibit poor workability, while ratios below 1:1 tend to exhibit poor mechanical properties, e. g., strength, and hence are not preferred. Preferred ratios are on the order of about 1: 3 to about 6: 1 and preferably about 1: 1 to 4: 1.

In case the ionomer cements of the invention may be further cured by a polymerisation reaction, the cements may be polymerized in accordance with known techniques. At least one initiator is required for most polymerization methods such as those based on oxidation/reduction reactions and ultraviolet and visible light.

In case the cement contains a redox initiator, the amount of reducing agent and oxidizing agent should be sufficient to provide the desired degree of polymerization. Preferably, the mixed but unset cements of the invention contain a combined weight of about 0.01 to about 10%, more preferably about 0.2 to about 5%, and most preferably about 0.5 to about 5% of the reducing agent and oxidizing agent, based on the total weight (including water) of the mixed but unset cement components. The reducing agent or the oxidizing agent can be microencapsulated as described in U.S. Pat. No. 5,154,762. This will generally enhance shelf stability of the cement parts and if necessary permit packaging both the reducing agent and oxidizing agent together. Water-soluble and water-insoluble encapsulants can be employed. Suitable encapsulating materials include cellulosic materials as cellulose acetate, cellulose acetate butyrate, ethyl cellulose, hydroxymethyl cellulose and hydroxyethyl cellulose being preferred. Other encapsulants include polystyrene, copolymers of polystyrene with other vinylic monomers and polymethylmethacrylate, copolymers of methylmethacrylate with other ethylenically-unsaturated monomers. Preferred encapsulants are ethylcellulose and cellulose acetate butyrate. By varying the choice of encapsulant and the encapsulation conditions, the onset of curing can be tailored to start at times ranging from seconds to minutes. The ratio of amount of encapsulant to activator generally ranges from 0.5 to about 10 and preferably from about 2 to about 6. Suitable oxidizing agents (initiators) include cobalt (III) chloride, tert-butyl hydroperoxide, ferric chloride, hydroxylamine (depending upon the choice of reducing agent), perboric acid and its salts, and salts of a permanganate or persulfate anion. Preferred oxidizing agents are potassium persulfate, ammonium persulfate and hydrogen peroxide. Suitable reducing agents (activators) include ascorbic acid, cobalt (II) chloride, ferrous chloride, ferrous sulfate, hydrazine, hydroxylamine (depending upon the choice of oxidizing agent) oxalic acid, thiourea, and salts of a dithionite or sulfite anion. Preferred reducing agents include ascorbic acid and ferrous sulfate.

Photoinitiators can also be added to the cement. A photoinitiator should be capable of promoting polymerization of the polymerizable groups on exposure to light of a suitable wavelength and intensity. The photoinitiator preferably is sufficiently shelf-stable and free of undesirable coloration to permit its storage and use under typical dental conditions. Visible light photoinitiators are preferred. The photoinitiator preferably is water-soluble or water-miscible. Suitable visible light-induced and ultraviolet light-induced initiators include an alpha-diketone (e.g., camphorquinone) with or without additional hydrogen donors (such as sodium benzene sulfinate, amines and amine alcohols). The photoinitiator may be present in an amount sufficient to provide the desired rate of photopolymerization. This amount will be dependent in part on the light source, the thickness of the cement layer to be exposed to radiant energy and the extinction coefficient of the photoinitiator. Preferably, mixed but unset photocurable cements of the invention will contain about 0.01 to about 5%, more preferably from about 0.1 to about 2% photoinitiator, based on the total weight (including water) of the mixed but unset cement components.

Depending upon the application of the cement and the manner in which polymerization is achieved, various components of the cement compositions may be packaged differently. For example, in the case of a redox-based system, ingredients of the cement composition are divided into two separate packages--the first package containing the copolymer, comonomer, the initiator and water, and the second package containing the reactive filler and the activator. In another embodiment, the first package contains all solid materials (e.g., copolymer, comonomer, reactive filler and if desired, the reducing agent, and the second package contains water and if desired, the initiator. In the case of photo-initiation, the photo-initiator can be included in either the solid (e. g. paste) or liquid parts of the cement.

The cements of the present invention may further contain non-reactive fillers, solvents, pigments, nonvitreous fillers, free radical scavengers, polymerization inhibitors, reactive and nonreactive diluents e.g., 2-hydroxyethyl methacrylate (HEMA), hydroxypropyl methacrylate, surfactants (such as to enhance solubility of an inhibitor e. g., polyoxyethylene), coupling agents to enhance reactivity of fillers e.g.,3- (trimethoxysilyl) propyl methacrylate, and rheology modifiers.

Suitable non-reactive fillers may be selected from fillers currently used in dental restorative compositions. The filler should be finely divided and preferably has a maximum particle diameter less than about 100 µm and an average particle diameter less than about 10 µm. The filler may have a unimodal or polymodal (e.g., bimodal) particle size distribution. The filler can be an inorganic material. It can also be a crosslinked organic material that is insoluble in the polymerizable resin, and is optionally filled with inorganic filler. The filler can be radiopaque, radiolucent or non-radiopaque. Examples of suitable non-reactive inorganic fillers are naturally-occurring or synthetic materials such as quartz, nitrides such as silicon nitride, glasses derived from, for example Ce, Sb, Sn, Zr, Sr, Ba and Al, colloidal silica, feldspar, borosilicate glass, kaolin, talc, titania, and zinc glass, and submicron silica particles such as pyrogenic silicas. Examples of suitable non-reactive organic filler particles include filled or unfilled pulverized polycarbonates or polyepoxides. Preferably the surface of the filler particles is treated with a coupling agent in order to enhance the bond between the filler and the matrix. The use of suitable coupling agents include gamma-methacryloxypropyltrimethoxysilane, gamma-mercaptopropyltriethoxysilane, gamma-aminopropyltrimethoxysilane, and the like.

Suitable solvents or nonreactive diluents include alcohols such as ethanol and propanol. Suitable reactive diluents are alpha,beta unsaturated monomers for providing altered properties such as toughness, adhesion, and set time.

Suitable alpha,beta-unsaturated monomers may be water-soluble, water-miscible or water-dispersible. Water-soluble, water-miscible or water-dispersible acrylates and methacrylates such as methyl acrylate, methyl methacrylate, ethyl acrylate, ethyl methacrylate, propyl acrylate, propyl methacrylate, isopropyl acrylate, isopropyl methacrylate, 2-hydroxyethyl acrylate, 2-hydroxyethyl methacrylate (HEMA), hydroxypropyl acrylate, hydroxypropyl methacrylate, tetrahydrofurfuryl acrylate, tetrahydrofurfuryl methacrylate, glycidyl acrylate, glycidyl methacrylate, the diglycidyl methacrylate of bis-phenol A ("bis-GMA"), glycerol mono-and di- acrylate, glycerol mono- and di- methacrylate, ethyleneglycol diacrylate, ethyleneglycol dimethacrylate, polyethyleneglycol diacrylate (where the number of repeating ethylene oxide units vary from 2 to 30), polyethyleneglycol dimethacrylate (where the number of repeating ethylene oxide units vary from 2 to 30 especially triethylene glycol dimethacrylate ("TEGDMA"), neopentyl glycol diacrylate, neopentylglycol dimethacrylate, trimethylolpropane triacrylate, trimethylol propane trimethacrylate, mono-, di-, tri-, and tetra- acrylates and methacrylates of pentaerythritol and dipentaerythritol, 1,3-butanediol diacrylate, 1,3-butanediol dimethacrylate, 1,4-butanedioldiacrylate, 1,4-butanediol dimethacrylate, 1,6-hexane diol diacrylate, 1,6-hexanediol dimethacrylate, di-2-methacryloyloxethyl hexamethylene dicarbamate, di-2-methacryloyloxyethyl trimethylhexanethylene dicarbamate, di-2-methacryloyl oxyethyl dimethylbenzene dicarbamate, methylene-bis-2-methacryloxyethyl-4-cyclohexyl carbamate, di-2-methacryloxyethyl-dimethylcyclohexane dicarbamate, methylene-bis-2-methacryloxyethyl-4-cyclohexyl carbamate, di-1-methyl-2-methacryloxyethyl-trimethyl-hexamethylene dicarbamate, di-1-methyl-2-methacryloxyethyl-dimethylbenzene dicarbamate, di-1-methyl-2-methacryloxyethyl-dimethylcyclohexane dicarbamate, methylene-bis-1-methyl-2-methacryloxyethyl-4-cyclohexyl carbamate, di-1-chloromethyl-2-methacryloxyethyl-hexamethylene dicarbamate, di-1-chloromethyl-2-methacryloxyethyl-trimethylhexamethylene dicarbamate, di-1-chloromethyl-2-methacryloxyethyl-dimethylbenzene dicarbamate, di-1-chloromethyl-2-methacryloxyethyl-dimethylcyclohexane dicarbamate, methylene-bis-2-methacryloxyethyl-4-cyclohexyl carbamate, di-1-methyl-2-methacryloxyethyl-hexamethylene dicarbamate, di-1-methyl-2-methacryloxyethyl-trimethylhexamethylene dicarbamate, di-1-methyl-2-methacryloxyethyl-dimethylbenzene dicarbamate, di-1-methyl-2-metha-cryloxyethyl-dimethylcyclohexane dicarbamate, methylene-bis-1-methyl-2-methacryloxyethyl-4-cyclohexyl carbamate, di-1-chloromethyl-2-methacryloxyethyl-hexamethylene dicarbamate, di-1-chloromethyl-2-methacryloxyethyl-trimethylhexamethylene dicarbamate, di-1-chloromethyl-2-methacryloxyethyl-dimethylbenzene dicarbamate, di-1-chloromethyl-2-methacryloxyethyl-dimethylcyclohexane dicarbamate, methylene-bis-1-chloromethyl-2-methacryloxyethyl4-cyclohexyl carbamate, 2,2'-bis(4-methacryloxyphenyl)propane, 2,2'bis(4-acryloxyphenyl)propane, 2,2'-bis[4(2-hydroxy-3-methacryloxy-phenyl)]propane, 2,2'-bis[4(2-hydroxy-3-acryloxy-phenyl)propane, 2,2'-bis(4-methacryloxyethoxyphenyl)propane, 2,2'-bis(4-acryloxyethoxyphenyl)propane, 2,2'-bis(4-methacryloxypropoxyphenyl)propane, 2,2'-bis(4-acryloxypropoxyphenyl)propane, 2,2'-bis(4-methacryloxydiethoxyphenyl)propane, 2,2'-bis(4-acryloxydiethoxyphenyl)propane, 2,2'-bis[3(4-phenoxy)-2-hydroxypropane-1-methacrylate]propane,and 2,2'-bis[3(4-phenoxy)-2-hydroxypropane-1-acryalte]propane, may be mentioned. Other suitable examples of polymerizable components are isopropenyl oxazoline, vinyl azalactone, vinyl pyrrolidone, styrene, divinylbenzene, urethane acrylates or methacrylates, epoxy acrylates or methacrylates and polyol acrylates or methacrylates.

Mixtures of alpha,beta-unsaturated monomers can be added if desired. Preferably, the mixed but unset cements of the invention will contain a combined weight of about 0.5 to about 40%, more preferably about 1 to about 30%, and most preferably about 5 to 20% water, solvents, diluents and alpha,beta-unsaturated monomers, based on the total weight (including such water, solvents, diluents and alpha,beta-unsaturated monomers) of the mixed but unset cement components.

An example of a suitable free radical scavanger is 4-methoxyphenol. An example of a suitable inhibitor is hydroxytoluene or butylated hydroxytoluene (BHT). The amount of inhibitor may be selected from 0.001 to 2% and preferably from 0.02 to 0.5% based on the total weight of the copolymer/comonomer/water mixture.

The transition metal complex surface modified reactive particulate filler may be used in a dental ionomer cement. Two major classes of such cements may be distinguished. The first class relates to conventional glass ionomers employing as their main ingredients a homopolymer or copolymer of an alpha,beta-unsaturated carboxylic acid (e.g., poly acrylic acid, copoly (acrylic, itaconic acid), etc.), a modified particulate reactive filler such as modified fluoroaluminosilicate glass, water, and a chelating agent such as tartaric acid. Such dental ionomer cements may be supplied in powder/liquid formulations that are mixed just before use. The mixture will undergo self-hardening in the dark due to an ionic reaction between the acidic groups of the polycarboxylic acid and cations leached from the glass as well as the comlexing reaction of the transition metals with the the acidic groups of the polycarboxylic acid. The second major class relates to resin-modified glass ionomer cements. Like a conventional glass ionomer, a resin-modified glass ionomer cement employs a modified particulate reactive filler obtainable according to the process of the present invention, whereby the organic portion of an resin-modified glass ionomer cements is different. In one type of resin-modified glass ionomer cement, the polycarboxylic acid is modified to replace or end-cap some of acidic repeating units with pendent curable groups and a photoinitiator is added to provide a second cure mechanism, e.g., as in US-A 5,130,347. Acrylate or methacrylate groups may be employed as the pendant curable group. A redox cure system can be added to provide a third cure mechanism, e.g., as in US-A 5,154,762. In another type of resin-modified glass ionomer cement, the cement includes a polycarboxylic acid, an acrylate or methacrylate-functional monomer and a photoinitiator, e.g., as in Mathis et al., "Properties of a New Glass lonomer/Composite Resin Hybrid Restorative", Abstract No. 51, J. Dent Res., 66:113 (1987) and as in US-A 5,063,257, US-A 5,520,725, US-A 5,859,089 and US-A 5,962,550. Various monomer-containing or resin-containing cements are also shown in US-A 4,872,936, US-A 5,227,413, US-A 5,367,002 and US-A 5,965,632. Resin-modified glass ionomer cements may be formulated as powder/liquid or paste/paste systems, and contain water as mixed and applied. They harden in the dark due to the ionic reaction between the acidic groups of the polycarboxylic acid and cations leached from the glass as well as the comlexing reaction of the transition metals with the the acidic groups of the polycarboxylic acid. Moreover, resin-modified glass ionomer cements also cure on exposure of the cement to light from a dental curing lamp.

The invention will now be further illustrated by the following Examples. All percentages refer to percentages by weight unless stated otherwise.

### Examples

### Example 1: Immobilisation of amino-ligands on the glass surface

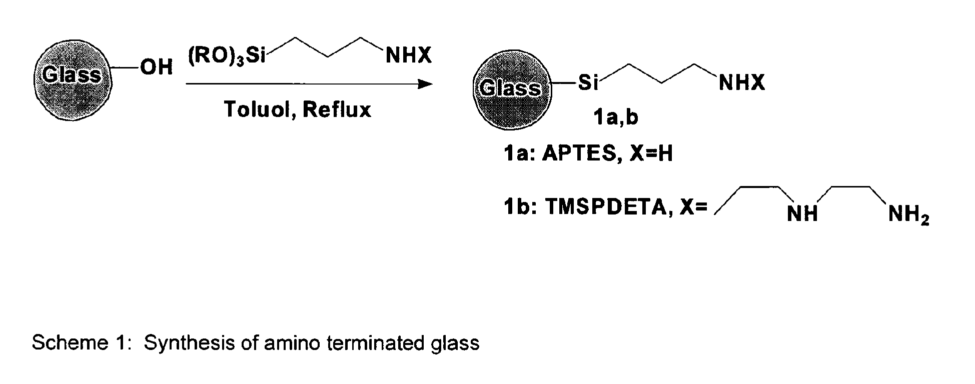

### APTES silaneted glass (1a)

To a suspension of 85g of a predried strontium aluminosilicate glass in 200ml of dry toluene was added 60ml (56.9g) of aminopropyltriethoxysilane (APTES). The mixture was stirred under reflux overnight and additionally stirred at room temperature for further 8h to complete the reaction. The slurry was filtered through a suction filter and washed with 3x50ml DCM, and 3x50ml petroleum ether (40/60). Drying at 50°C under 4mbar yielded a slightly yellow coloured glass displaying pendant monoamino functions.

### TMSPDETA silaneted glass (1b)

To a suspension of 85g of a predried strontium aluminosilicate glass in 240ml of dry toluene was added 60g of (3-trimethoxysilylpropy)diethylenetriamine (TMSPDETA). The mixture was stirred under reflux overnight and additionally stirred at room temperature for further 8h to complete the reaction. The slurry was filtered through a suction filter and washed with 3x50ml DCM (dichloromethane), and 3x50ml petroleum ether (40/60). Drying at 50°C under a reduced pressure of 4 mbar yielded a slightly yellow coloured glass displaying pendant diethylenetriamino functions.

### Example 2

### Preparation of glasses modified by transition metal TMSPDETA complexes

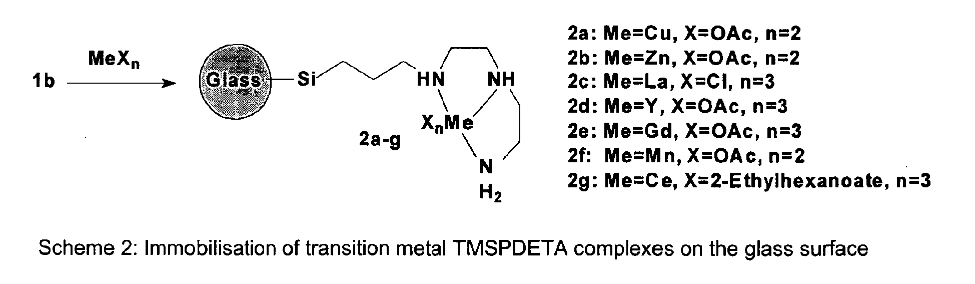

### Copper modified glass (2a)

A slurry of 23 g of the TMSPDETA modified strontium aluminosilicate glass **(1b)** in 50ml of MeOH was stirred with a solution of 34mg of Cu(II) acetate in 10ml of MeOH overnight at RT (room temperature). The glass was filtered over a P3-frit and washed excessively with MeOH (methanol) in order to remove any mobile metal ions. Drying at 50°C for 24h yielded a blueish-green coloured Cu(II) modified glass **2a.**

### Zinc modified glass (2b)

A slurry of 20 g of the TMSPDETA modified strontium aluminosilicate glass **(1b)** in 50ml of MeOH was stirred with a solution of 20mg of zinc acetate in 10ml of MeOH overnight at RT. The glass was filtered over a P3-frit and washed excessively with MeOH in order to remove any mobile metal ions. Drying at 50°C for 24h yielded a slightly yellow coloured zinc modified glass **2b**.

### Lanthanum modified glass (2c)

A slurry of 20 g of the TMSPDETA modified strontium aluminosilicate glass **(1b)** in 50ml of MeOH was stirred with a solution of 37mg of lanthanum chloride hydrate in 10ml of MeOH overnight at RT. The glass was filtered over a P3-frit and washed excessively with MeOH in order to remove all not immobilized metal ions. Drying at 50°C for 24h yielded a slightly yellow coloured lanthanum modified glass 2c.

### Yttrium modified glass (2d)

A slurry of 20 g of the TMSPDETA modified strontium aluminosilicate glass **(1b)** in 50ml of MeOH was stirred with a solution of 26mg of yttrium acetate in 10ml of MeOH overnight at RT. The glass was filtered over a P3-frit and washed excessively with MeOH in order to remove any mobile metal ions. Drying at 50°C for 24h yielded a slightly yellow coloured yttrium modified glass **2d**.

### Gadolinium modified glass (2e)

A slurry of 20 g of the TMSPDETA modified strontium aluminosilicate glass (**1b**) in 50ml of EtOH (ethanol) was stirred with a solution of 26mg of gadolinium acetate hydrate in 10ml of EtOH overnight at RT. The glass was filtered over a P3-frit and washed excessively with EtOH in order to remove any mobile metal ions. Drying at 50°C for 24h yielded a gadolinium modified glass 2e.

### Manganese modified glass (2f)

A slurry of 20 g of the TMSPDETA modified strontium aluminosilicate glass **(1b)** in 50ml of MeOH was stirred with a solution of 18mg of Mn(II) acetate in 10ml of MeOH overnight at RT. The glass was filtered over a P3-frit and washed excessively with MeOH in order to remove any mobile metal ions. Drying at 50°C for 24h yielded a slightly brownish coloured manganese modified glass **2d.**

### Cerium modified glass (2g)

A slurry of 20 g of the TMSPDETA modified strontium aluminosilicate glass (**1b**) in 55ml of MeOH was stirred with a solution of 60mg of Ce(III) 2-ethylhexanoate in 10ml of MeOH overnight at RT. The glass was filtered over a P3-frit and washed excessively with MeOH in order to remove any mobile metal ions. Drying at 50°C for 24h yielded a slightly brownish coloured cerium modified glass **2e**.

### Example 3

### Preparation of GIC formulations containing modified glasses

Based on each of the above mentioned glasses modified by amine or transition metal complex, experimental ionomer cements were prepared and compared to a formulation containing untreated strontium aluminosilicate glass.

Experimental ionomer cements contain an experimental glass ionomer powder and an experimental glass ionomer liquid. The experimental glass ionomer powders contain 72% glass modified by amine or transition metal complexes and 28% of powdered polyacrylic acid. powders were mixed with an experimental glass ionomer liquid containing water (59%), PAA (32%) and tartaric acid (9%) in the ratio 3.7 : 1. These formulation were investigated with respect to their working time, setting time, compressive strength and flexural strength. The results are listed in the following Table 1.

**Table 1**

| Formulation | **F1a** | **F1b** | **F2a** | **F2b** | **F2c** | **F2d** | **F2e** | **F2f** | **F2g** | **Reference** |
|---|---|---|---|---|---|---|---|---|---|---|
| Glass contained | **1a** | **1b** | **2a** | **2b** | **2c** | **2d** | **2e** | **2f** | **2g** | **Untreated glass** |
| WT[min] | 5.0 | 4.3 | 3.8 | 3.9 | nd | nd | 3.8 | 3.2 | 3.3 | 2.5 |
| ST [min] | 2.3 | 2.5 | 2.2 | 2.3 | nd | nd | nd | nd | 2.5 | 2.3 |
| CS [MPa] | 187 | 176 | 192 | 188 | 217 | 196 | 193 | 246 | 218 | 196 |
| FS [MPa] | 40 | 41 | 46 | 34 | 40 | 34 | 45 | 39 | 48 | 36 |

| | | | | | | | | | | |
|---|---|---|---|---|---|---|---|---|---|---|
| WT = Working Time | | | | | | | | | | |
| ST = Setting Time | | | | | | | | | | |
| CS = Compressive Strength | | | | | | | | | | |
| FS = Flexural Strength | | | | | | | | | | |

### Example 4

### Synthesis of [N-(aminoethyl)-trimethoxysilylpropylamine]-copper(II)-diacetate

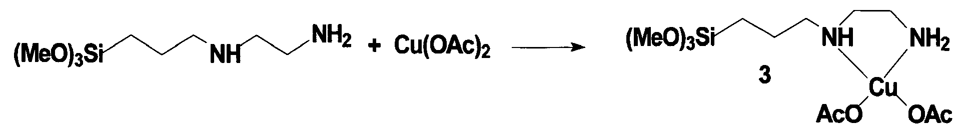

To a solution of 1.81g (10mmol) of Cu(II)acetate in 100ml of dry MeOH, a solution of 2.22g (10mmol) of [3-(2-aminoethyl)aminopropyl]-trimethoxysilane in 20ml of dry MeOH was dropped. The mixture was stirred for 30min at room temperature. Then the solvent was removed under vacuum and the desired trimethoxysilyl-functionalized copper - diamine complex 3 was obtained as dark blue highly viscous oil. IR: 2939, 2841 (CH₂, CH₃) 1562, 1387 (OAc), 1190 (Si-CH₂), 1075 (Si-OCH₃)

### Immobilisation of [N-(aminoethyl)-trimethoxysilypropylamine]-copper(II)-diacetate

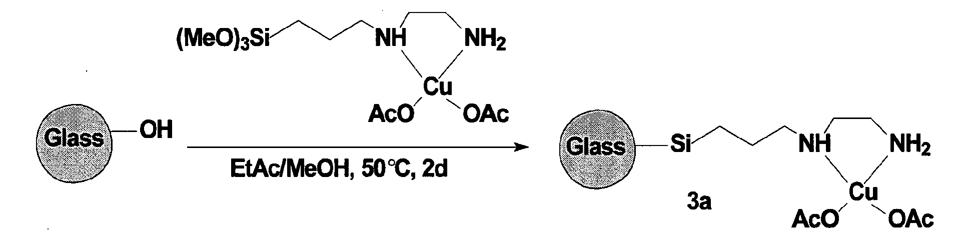

43g of predried strontium aluminosilicate glass was mixed with 50mL of dry ethyl acetate. To this slurry a solution of 1.03g (2.5mmol) of [N-(aminoethyl)-trimethoxysilypropylamine]-copper(II)-diacetate in 20ml of MeOH was added. This slurry was stirred at 50°C for two days. The glass was filtered over a P3-frit and washed excessively with MeOH in order to remove any mobile copper diamine complex. Drying at 50°C for 24h yielded in the bluish-green coloured Cu(II)-diamine modified glass **3a**

### Example 5

### Synthesis of [N-(aminoethyl)-trimethoxysilypropylamine]-gadolinium(III)-triacetate

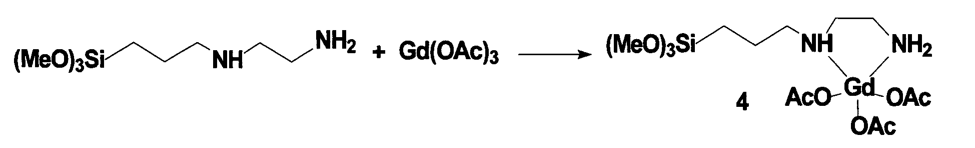

To a solution of 3.34g (10mmol) of Gd(III)acetate in 100ml of dry EtOH a solution of 2.22g (10mmol) of [3-(2-aminoethyl)aminopropyl]-trimethoxysilane in 20ml of dry EtOH was dropped. The mixture was stirred for 30min at room temperature. Then the solvent was removed under vacuum and the desired trimethoxysilyl functionalized gadolinium - diamine complex **4** was obtained as colourless highly viscous oil.
IR: 2971, 2928, 2838 (CH₂, CH₃) 1544, 1406 (OAc), 1188 (Si-CH₂), 1074 (Si-OCH₃)

### Immobilisation of [N-(aminoethyl)-trimethoxysilypropylamine]-gadolinium(III)-triacetate

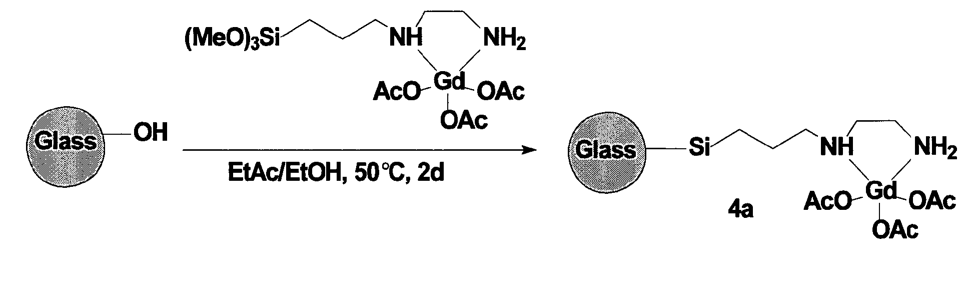

40g of predried strontium aluminosilicate glass was mixed with 100mL of dry ethyl acetate. To this slurry a solution of 1.30g (2.5mmol) of [N-(aminoethyl)-trimethoxysilypropylamine]-gadolinium(III) triacetate in 50ml of EtOH was added. This slurry was stirred at 50°C for two days. The glass was filtered over a P3-frit and washed excessively with MeOH in order to remove any mobile gadolinium diamine complex. Drying at 50°C for 24h yielded the colourless Gd(III)-diamine modified glass **4a**.

### Preparation of glass ionomer cement formulations containing modified glasses

From each above mentioned glasses modified by an transition metal complex, experimental ionomer cements were prepared and compared to a formulation containing untreated strontium aluminosilicate glass.

Experimental ionomer cements contain an experimental glass ionomer powder and an experimental glass ionomer liquid. The experimental glass ionomer powder containing 72% glass modified by amine or transition metal complex and 28% of powdered polyacrylic acid were mixed with an experimental glass ionomer liquid containing water (59%), PAA (32%) and tartaric acid (9%) in the ratio 3.7 : 1. These formulation were investigated with respect to their working time, setting time, compressive strength and flexural strength. The results are listed in table 1.

**Table 1**

| **Formulation** | **F3a** | **F4a** | **Reference** |
|---|---|---|---|
| **Glass contained** | **3a** | **4a** | **Untreated glass** |
| WT [min] | 1.8 | 3.0 | 2.5 |
| ST [min] | 1.1 | 2.0 | 2.3 |
| CS [MPa] | 233 | 213 | 196 |
| FS [MPa] | 46 | 40 | 36 |

| | | | |
|---|---|---|---|
| WT = Working Time | | | |
| ST = Setting Time | | | |
| CS = Compressive Strength | | | |
| FS = Flexural Strength | | | |

As shown by the above examples and comparative examples, the use of a particulate reactive filler modified according to the present invention provides a dental ionomer cement having improved mechanical properties, in particular improved compressive strength and flexural strength, while at the same time having excellent working and setting times.

## Claims

1. A process for the modification of a particulate reactive filler for a dental ionomer cement, comprising
(a) providing a particulate reactive filler; and
(b1) treating the surface of the particulate reactive filler with a surface modifying agent for obtaining a surface modified particulate reactive filler displaying ligand groups for a transition metal; and
(b2) treating the surface modified particulate reactive filler with an agent containing the transition metal for complexing the transition metal with the ligand groups displayed on the surface of the surface modified particulate reactive filler; and/or
(c) treating the surface of the particulate reactive filler with a surface modifying transition metal complex which is a reaction product of a surface modifying agent and a transition metal precursor compound,
for obtaining a transition metal complex surface modified particulate reactive filler for a dental ionomer cement.

2. The process according to claim 1 wherein the reactive filler contains a glass.

3. The process according to claim 1 or 2, wherein the reactive filler is selected from calcium alumino silicate glass, calcium alumino fluorosilicate glass, calcium aluminumfluoroborosilicate glass, strontium aluminum silicate glass, strontium alumino fluorosilicate glass, strontium aluminumfluoroborosilicate glass.

4. The process according to any one of the preceding claims wherein the particulate reactive filler has a an average particle size of from 0.005 to 100 µm.

5. The process according to any one of the preceding claims wherein the particulate reactive filler has a an average particle size of from 0.01 to 40 µm.

6. The process according to any one of the preceding claims wherein the surface modifying agent contains a hydrolyzable organofunctional silicon compound.

7. The process according to claim 5 wherein the hydrolyzable organofunctional silicon compound is a compound of one of the following formulae (I), (II) and (III), or a hydrolysis product thereof
XₙR₃₋ₙSiL (I)
XₙR₂₋ₙSiL'L" (II)
XₙSiL'L"L"' (III)
wherein
X represents a hydrolyzable group;
R represents an alkyl, cycloalky, cycloalkylalkyl, aralkyl or aryl group
L, L', L", and L"' which may be the same or different represent independent from each other an organic group containing hetero atoms capable of coordinating to the transition metal;
n is an integer ≥1,
whereby the sum of X, R, L, L', L", and L"' is 4 for each of formula (I), (II), and (III).

8. The process according to claim 7, wherein X is a halogen atom or OR, wherein R is as defined in claim 7.

9. The process according to claim 7 or 8, wherein R is an alkyl group.

10. The process according to claim 6, 7 or 8, wherein L, L', L", and L"' contain nitrogen atoms, oxygen atoms, sulfur and/or phosphorous atoms capable of binding to the transition metal.

11. The process according to any one of claims 8 or 9, wherein L, L', L", and L'" may be represented by the following formula:
-[(CH₂)ₒZ]_{q}(CH₂)ₚZR'
wherein
the Zs which may be the same or different and are independent from each other, represent -NR'- , -O-, S or PR'
R' represents independently a hydrogen atom, an alkyl group, a cycloalkyl group, an cycloalkylalkyl group, an aralkyl group or an aryl group,
o and p, which are independent from each other, may be the same or different and represent an integer of from 1 to 6, and
q represents an integer of from 0 to 12.

12. The process according to any one of claims 8 or 9, wherein L, L', L", and L'" may be represented by the following formula:
-[(CH₂)ₒNR']_{q}(CH₂)ₚNR"R"'
wherein
R', R" and R"', which are independent from each other, may be the same or different and represent a hydrogen atom, an alkyl group, a cycloalkyl group, an cycloalkylalkyl group, an aralkyl group or an aryl group,
o and p, which are independent from each other, may be the same or different and
represent an integer of from 1 to 6, and
q represents an integer of from 0 to 12.

13. The process according to any one of claims 8 or 9, wherein L, L', L", and L'" may be represented by the following formula:
-[(CH₂)ₒZ]_{q}(CH₂)ₚZR'
wherein
R' represents a hydrogen atom, an alkyl group, a cycloalkyl group, an cycloalkylalkyl group, an aralkyl group or an aryl group,
Z represents an oxygen atom or a sulfur atom,
o and p, which are independent from each other, may be the same or different and represent an integer of from 1 to 6, and
q represents an integer of from 0 to 12.

14. The process according to any one of the preceding claims wherein the surface modifying agent contains a compound selected from the group of aminopropyltrimethoxysilane, aminopropylmethyldimethoxysilane, aminopropyldimethylmethoxysilane, aminopropyltriethoxysilane (APTES), aminopropylmethyldiethoxysilane, aminopropyldimethylethoxysilane, 2-(aminoethyl)-3-aminopropyltrimethoxysilane (AEPTMS), 2-(aminoethyl)-3-aminopropyldimethoxymethylsilane, 2-(aminoethyl)-3-aminopropyldimethylmethoxysilane, 2-(aminoethyl)-3-aminopropyltriethoxysilane, 2-(aminoethyl)-3-aminopropyldiethoxymethylsilan, 2-(aminoethyl)-3-aminopropyldimethylethoxysilane, (3-trimethoxysilylpropyl)diethylenetriamine,(3-dimethoxymethylsilylpropyl)diethylenetriamine,(3-dimethylmethoxysilylpropyl)diethylenetriamine, (3-triethoxysilylpropyl)diethylenetriamine (TMSPDETA), (3-diethoxymethylsilylpropyl)diethylenetriamine, (3-dimethylethoxysilylpropyl)diethylenetriamine.

15. The process according to any one of the preceding claims wherein the transition metal is selected from the group of scandium, yttrium, lanthanum, cerium, praseodymium, neodymium, samarium, europium, gadolinium, terbium, dysprosium, holmium, erbium, thulium, ytterbium, lutetium, manganese, copper, silver, ruthenium, rhodium, palladium, zinc and iron.

16. The process according to any one of the preceding claims wherein the transition metal is selected from the group of yttrium, lanthanum, cerium, samarium, europium, gadolinium, terbium, holmium, ytterbium, lutetium, copper, and zinc.

17. The process according to any one of the preceding claims wherein the transition metal is an ion capable of forming a complex with a carboxyl group.

18. Reactive particulate filler for a dental ionomer cement obtainable according to the process according to any one of the preceding claims.

19. Dental ionomer cement comprising the reactive particulate filler according to claim 18.

20. The dental ionomer cement according to claim 19, which further comprises a polyacidic polymer selected from polyacrylic acid, polymethacrylic acid, polymaleic acid and polyitaconic acid or copolymers of acrylic acid, methacrylic acid, maleic acid and itaconic acid.

21. The dental ionomer cement according to claim 20 or 21, which further comprises tartraic acid, a non-reactive filler such as aerosil, and pigments.

22. The dental ionomer cement according to any one of claims 19 to 21, which is a resin modified dental ionomer cement.

23. Use of the reactive particulate filler for a dental ionomer cement obtainable according to the process according to any one of claims 1 to 16 for the preparation of a dental composition.
